# EUROPEAN PATENT SPECIFICATION

(11) **EP 2 841 002 B1**
(45) Date of publication and mention of the grant of the patent: **24.03.2021**
(21) Application number: 13781116.2
(22) Date of filing: 23.04.2013
(51) Int. Cl.: A61F 2/00, A61F 2/36

(54) **PACKAGING FOR MEDICAL ARTICLES SUCH AS A SIZE VARYING SERIES OF ORTHOPEDIC IMPLANTS**
VERPACKUNG FÜR MEDIZINISCHE GEGENSTÄNDE WIE ETWA EINE GRÖSSENVARIIERENDE SERIE VON ORTHOPÄDISCHEN IMPLANTATEN
EMBALLAGE POUR ARTICLES MÉDICAUX TELS QU'UNE SÉRIE À TAILLE VARIABLE D'IMPLANTS ORTHOPÉDIQUES

(30) Priority: 27.04.2012 US 201213457798
(43) Date of publication of application: 04.03.2015
(73) Proprietor: Bemis Healthcare Packaging, Inc., Oshkosh WI 54901 (US)
(72) Inventor: KINYON, Tad Ray, Elysian, MN 56028 (US)
(74) Representative: Vossius & Partner Patentanwälte Rechtsanwälte mbB
(86) International application number: PCT/US2013/037767
(87) International publication number: WO 2013/163160

(56) References cited:
- EP-A1- 0 523 895
- NL-A- 6 708 222
- US-A- 3 353 664
- US-A- 4 921 096
- US-A- 5 842 567
- US-A1- 2005 098 460
- US-A1- 2007 295 620
- US-A1- 2011 155 592

## Description

### Background of the Invention

The packaging of the femoral stem component used in the orthopedic hip arthroplasty procedure has traditionally involved the use of inserts to stabilize the stem component within the package and these inserts have been tailored to the size of a particular stem component. Stem components are currently available in a substantial number of sizes with some manufacturers offering as many as ten sizes in order to better meet the needs of individual patients.

The traditional packaging of sterile medical devices such as implants including stem components has involved a system of an inner tray within an outer tray. Each tray is typically an open mouthed cavity with a peripheral rim to which a film is adhesively adhered to create a sealed package. The outer tray simply contains the inner tray which in turn contains the medical device, commonly stabilized within the tray with closed cell foam pieces. The pieces of foam are commonly selected to have configurations adapted to the particular device being packaged. Thus in the case of stem components different pieces of foam are required in progressing across the size range of such components. The two tray system provides some assurance that if the integrity of the outer tray is breached in shipping and handling, the sterility of the packaged medical device is preserved by the inner tray.

This two tray system has some disadvantages. The foam used for stabilization within the inner tray is friable and, particularly with orthopedic implants with roughened surfaces to enhance bonding to living tissue, typically bone, it has been observed to abrade, creating a particulate contaminate. In addition, because the peripheral rim of the inner tray typically carries residual adhesive after the removal of the lid stock, it may not be placed on the surgical tray adjacent to the surgeon implanting the device. Consequently, the medical device must be fully removed from its protective packaging well before its use and is thus exposed to damage and being splashed with bodily fluids and tissue while awaiting implantation.

Thus there are benefits to be gained from a packaging approach in which a single package can be used across the size range of at least a single line of femoral stem components of a given design or from a single manufacturer. There are further benefits to be obtained from a complete package which can be removed from an inner tray and placed on a surgical tray thus providing protection for the packaged component until it is used and providing a convenient manner of presenting the component to the surgical team A packaging for medical articles is known from the document EP-A-0523895.

### Summary of the invention

The present invention involves a package which is adapted to securely hold medical articles such as medical instruments or implants e.g. orthopedic implants such as any of a series of femoral stem components of artificial hip joints which have a dimension which does not vary greatly over a significant size range. This package is configured to provide a clearance which captures and closely approximates a minimally varying dimension of a medical article

The present invention relates to a rigid synthetic polymer package for medical articles as set forth in the appended claims.

In the present invention a rigid, synthetic e.g. thermoplastic polymer package is provided having (A) a rigid base and (B) a cover having at least two compartments connected by a connecting hinge. The base and cover may also be connected by a peripheral hinge. The first compartment has a first interior surface and opposing first exterior surface where the first interior surface has one or more cavities whereby this first interior surface is adapted for movement restraining contact with a first portion of a medical article which has a fixed or minimally varying dimension. The second compartment has a second interior surface and opposing second exterior surface where the second interior surface has one or more cavities whereby this second interior surface is adapted for contact, preferably movement restraining contact, with a second portion of a medical article. The connecting hinge connects the first and second compartments to each other and provides for manual movement of the first compartment relative to the position of the second compartment to rotate the first interior surface away from the second interior surface and the opposite rotational movement is restricted or prevented e.g. the first interior surface cannot be rotated to cover the second interior surface.

One convenient way to provide the aforementioned clearance which captures and closely approximates a minimally varying dimension in a package for an orthopedic implant such as a hip implant is to provide a fairly flat rectangular package with three distinct but interconnected cavities, each of which accommodates one of the three distinct elements of a femora! stem component. In this regard, the typical stem component is comprised of a lower stem which is a long generally cylindrical portion adapted to be inserted into a femur, an angled shaft which is a short cylindrical portion adapted to be inserted into the femoral head or ball and a body which is a transition portion, also adapted to be inserted into the femur, which connects the short and long cylindrical portions. One edge of this body is typically just an extension of one edge of the lower stem and the other edge proceeds outward at an acute angle from the main axis of the lower stem. The angled shaft then extends outward from the end of the body distal from the lower stem at an acute angle to the main axis of the lower stem which is typically greater than the acute angle between the one edge of body and said axis. These three portions typically have about the same thickness such that they all extend about the same distance in a z direction where x and y directions extend along the length and width of the femoral stem component. In a preferred embodiment the largest member of the series femoral stem components for which the package is adapted just fits within the package with smaller members fitting in with some overall clearance.

One convenient approach is a package having a first compartment having first and second cavities connected by an integrally formed hinge to a second compartment having a cavity. In this approach the capturing clearance is provided by the cooperation of the second compartment's cavity adapted to contain the lower stem and the first compartment's cavity adapted to contain the body. In this approach, a communication port between these two cavities is sized to prevent the entrance of the body from the first compartment into the second compartment's cavity for the lower stem; and in the first compartment a wall of the first cavity adapted to contain the body distal from this port limits the motion of the body toward the second cavity adapted to contain the angled shaft. For this approach the cavities for the lower stem in the second compartment and the angled shaft in the first compartment may readily accommodate the full size range of the series with a significantly greater clearance. In a preferred embodiment, this distal wall of the body cavity is at an obtuse angle to the main axis of the lower stem cavity of the package. In a particularly preferred embodiment the body cavity has a wall which is a continuation of a wall of the lower stem cavity and the obtuse wall begins at the end of this continuation wall distal from the port between the lower stem cavity and the body cavity. In a particularly preferred embodiment the package is provided with two sets of cavities to accommodate the angled shaft and lower stem parts of the femoral stem component with both sets of cavities communicating with a common cavity for accommodating the body part. In an especially preferred version of this approach this common cavity for accommodating the body has two walls parallel to the main axis of the lower stem cavities, which are themselves parallel to each other, with each wall terminating at its end distal to the ports communicating with the lower stem cavities in an obtuse wall which in turn extends to one of the ports communicating with one of the cavities for accommodating the angled shaft.

One convenient approach to creating a package for a series of orthopedic implants such as femoral stem components is to create two flat generally rectangular tray components which are mirror images of each other, with each carrying one half of the three portions to adapted accommodate the lower stem, the body and the angled shaft, respectively, of the femoral stem component. These tray components are provided with means which reversibly lock them together so as to create the three communicating cavities which will accommodate the series of femoral stem components. In a preferred embodiment, each tray component is provided with a hinge located at approximately the juncture between the cavity for the lower stem and the cavity for the body which extends across the width of the tray component to define an upper tray section and a lower tray section. The two mating lower tray sections are provided with locking means which function to hold them together and the two mating upper tray sections are provided with locking means which function to reversibly hold them together. The two hinges and the reversible upper tray section locking means function to allow the two upper tray sections to be rotated away from each other. In an especially preferred embodiment the two tray components each carry two cavities for the lower stem and two cavities for the angled shaft and the two oblique walls discussed above.

In another approach, mating trays need not be utilized, but instead a generally flat rigid base is employed with a cover similar to the tray above but having deeper cavities in its first and second compartments. This base and cover may be either separate components or they themselves may be connected by a hinge at one of the peripheral edges of the cover.

### Brief Description of the Drawings

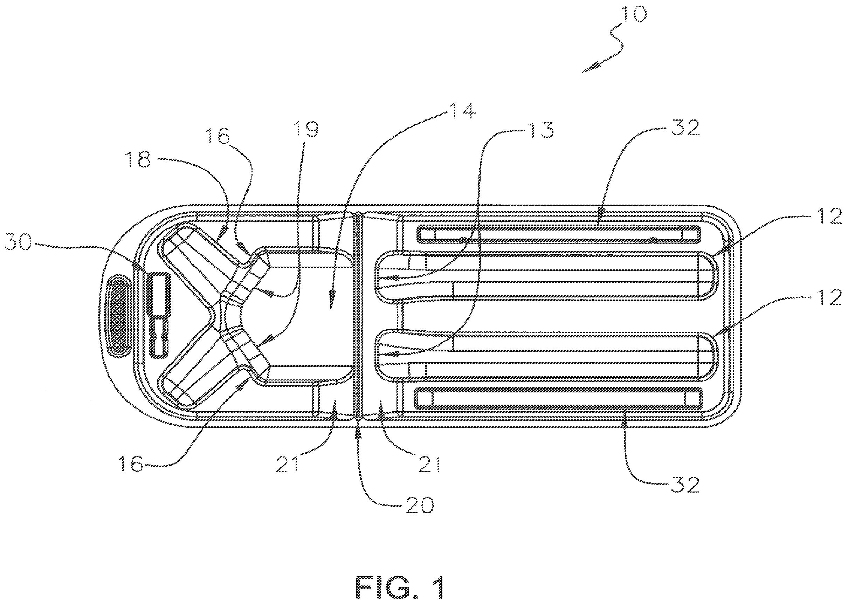

Fig. 1 is a plan view of one of the two tray components 10 which may be a cover or base showing its longer hemi-tubular cavities 12, the ports 13 leading from the cavities 12 to its transition cavity 14, the oblique walls 16 of its transition cavity 14, the ports 19 leading from cavity 14 to its shorter hemi-tubular cavities 18, its hinge 20, its reversible upper tray section locking means 30 and its reversible lower tray section locking means 32.

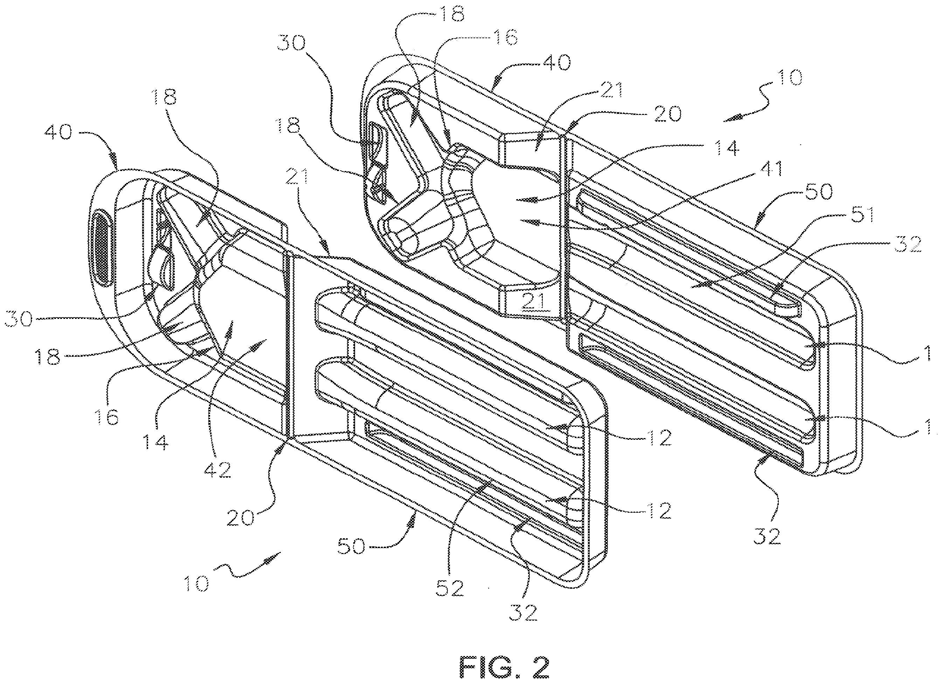

Fig. 2 is a perspective view of the two tray components 10 facing each other such that they can be assembled by forcing them together to form a package for a femoral stem component. In addition to all the elements illustrated in Fig. 1, also shown are the upper tray sections 40 and the lower tray sections 50.

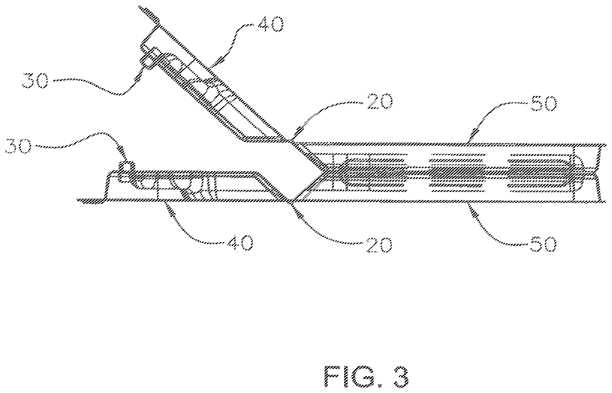

Fig. 3 is a perspective view of the two tray components 10 assembled together showing how the upper tray sections can rotate away from each other via the hinges 40 while the lower tray sections 50 remain affixed to each other.

### Detailed Description of the invention

The present invention involves the design of packaging for medical articles such as medical instruments such cutting implements or implants such as orthopedic implants e.g. hip stems, shoulder stems, fixation devices which may be a length of e.g. stainless steel which is used to stabilize fractured bone, or other medical articles which may have abrasive surfaces or surfaces which have coatings or other materials for which contamination or surface removal by abrasion is a concern. In particular the invention may be used for a series of components e.g. shoulder or femoral stem components of various sizes e.g. for artificial joints such as for shoulders or hips, which components have a dimension which does not greatly vary over the size range of the series. The allowed variation in this dimension is such that the package can be designed to just accommodate this dimension for the largest member of the series and yet still accommodate the same dimension of the smallest member of this series without allowing undue motion of this smallest member. The package of the present invention can allow some range of motion to the smallest member of the series so long as this packaged component can still pass the shipping tests to which the manufacturers of medical articles, such as implants, routinely subject such products. This depends on the configuration and weight of the particular component being packaged and tested. In a preferred embodiment the packaging for a given series of stem components will provide a range of clearances for this dimension which ranges from essentially zero to 0,375 inches. The invention also involves the concept of designing such a common package with a clearance which captures this minimally varying dimension but also accommodates a range of sizes in the other dimensions of the medical articles e.g. stem components such as femoral stem components.

It is advantageous to construct the packaging such that the surfaces which contact the surfaces of the stem component have a high abrasion resistance, In this regard, medical articles such as orthopedic implants like stem components are typically constructed with a body portion which has a fairly rough surface to promote adhesion to the bones into which they are implanted. One type of surface with advantageous abrasion resistance is based upon thermoplastic aromatic polyether polyurethane. Deerfield Urethanes, a subsidiary of Bayer, markets suitable thermoplastic aromatic polyether polyurethane films under the trademark Dureflex® with grade PT9400 being particularly suitable, It is also advantageous to use this polyurethane surface to protect to the polished treated surfaces typical of orthopedic implants e.g. femoral stem components such as portions of the angled shaft.

It is also advantageous to construct the packaging out of materials which can be readily thermoformed into packages of suitable rigidity to support the largest component of the series for which the packaging is designed. The typical packaging material for medical implants including femoral stem components has been films of polyethylene terephthalate glycol (PETG) because they have adequate rigidity and mechanical strength and good thermal formability. However, it is desirable to have greater abrasion resistance than this material offers. It is convenient to laminate these films to films of thermoplastic aromatic polyether polyurethane and configure the packaging such that it is the polyurethane surface which faces the surfaces of the medical article e.g. stem component. The laminate is conveniently formed by melt laminating the polyurethane to the PETG. A preferred PETG for this lamination is Eastman's Eastar 6763 PETG resin. The thicknesses for both films should be compatible with both thermoforming the package configuration and providing adequate mechanical strength. A convenient range for the polyurethane is between about 0.01 and 0.025 inches while for the PETG it is between about 0.015 and 0.04 inches.

The packages with which the present invention is concerned can be conveniently designed using computer-aided design (CAD). One approach is to create three dimensional depictions of all the components in a given series such as the size range offered by a given manufacturer e.g. for stem components and then overlay them to determine the location and size of the minimally varying dimension. Commonly, for orthopedic implants such as stem components this is the length of the body portion measured along its outside edge which is a continuation of an outside edge of the lower stem component. Then a package can be designed which provides a clearance which closely approximates minimally varying this dimension and also accommodates the largest size component in the series e.g. the largest femoral component.

One approach to the packaging of a series of femoral stem components is illustrated in Fig. 1-3. In this approach a base and a cover which may be two mirror image trays 10 are provided and are joined to each other in a face to face manner as illustrated in Fig. 2. Each tray 10 carries a cavity 12 to accommodate the lower stem and first and second cavities 14 and 18 to accommodate the, body and angled shaft portions respectively of a stem component. The cavities 12 and 18 are hemi-tubular such that when two trays are joined in a face to face manner they provide tubular cavities to accommodate the somewhat cylindrical elements e.g. of the femoral stem component. These cavities 12 and 18 communicate with the cavity 14 which is designed to accommodate the body portion of the stem component via ports 13 and 19, respectively. This cavity 14 is provided with two oblique walls 16 which are at an oblique angle to the main axis of the tray 10 or the cavities 12. Either of the ports 13 and the oblique wall 16 directly opposite this port 13 in the direction of the main axis of the tray component 10 cooperate to capture the minimally varying dimension of the body of the stem component. The port 13 is designed such that the portion of the body adjacent to the lower stem of the series e.g. of femoral stem components for which the package is designed is unable to enter this port 13. The distance from this port 13 to this oblique wall 16 approximates the length of the edge of the body portion which is a continuation of an outer edge of lower stem portion of e.g. the largest femoral stem component of the series for which the package is designed. Typically stem components such as femoral stem components carry an edge which proceeds at an oblique angle from the edge of the body portion which is a continuation of an outer edge of lower stem portion to the edge from which the angled shaft projects and it is this edge that the oblique wall 16 approximates. The trays 10 also each carry a hinge 20 which allows the package to be partially opened after assembly and two reversible closure means 30 and 32 to facilitate the assembly of the two trays 10 in a face to face manner.

The illustrated trays 10 are shown as each being symmetric about its main axis because this facilitates their production from a single mold. Any two tray components can then be placed face to face with their cavities facing inward to create an assembled package. However, non-symmetrical tray components could readily be designed to accommodate two different series of femoral stem components. In this case, separate molds would be required for the top and bottom tray components, but the number of sizes that the package could accommodate would be increased. Also, the base and cover or top and bottom trays may be connected by a peripheral hinge alone a top, bottom or side edge.

The package is provided with the ability to be partially opened to allow access to the packaged medical article such as a scalpel, catheter, drug coated implant, or an orthopedic implant e.g. a stem component while still providing some protection and support to this article. The hinges 20 divide each tray component 10 into an upper section or first compartment 40 having an interior surface 41 and exterior surface 42 and a lower section or second compartment 50 having an interior surface 51 and exterior surface 52 as illustrated in Fig. 2-3. The hinges 20 also allow the two upper sections 40 to be rotated away from each other as illustrated in Fig. 3. Thus the connecting hinges 20 (which may be integrally formed with the first and second compartments 40, 50) provide for manual movement of the first compartment 40 relative to the second compartment 50 to rotate a first interior surface 41 of a cover or upper tray from a second interior surface of a base or lower tray. Also, the first compartment of the tray cannot be rotated about an axis of the hinge 20 to a position where the first interior surface 41 of the first compartment covers the second interior surface 51 of the second compartment because of stops formed by hinge walls 21. The provision of separate closure means 30 for the upper tray section 40 and closure means 32 for the lower tray section 50 facilitates the partial opening.

This ability to partially open the package is viewed as a significant benefit to surgical teams e.g. a surgical team implanting an orthopedic article such as a femoral stem component. It allows them clean easy access to a medical article such as a stem component without having it exposed before it needs to be withdrawn from the protective packaging for use such as implantation. It also significantly reduces the risk of dropping the article in the course of removing it from its packaging. To extract a medical article e.g. the stem component from the partially opened package one must have a firm grip on it, while if the two tray components were not hinged for partial opening, but simply stripped from each other there is a chance the stem component would just fall from the packaging.

### Working Example

A prototype set of tray components 10 were thermoformed from a melt lamination of 0.0254 cm (0.01 in) thick Duraflex®PT 9400 aromatic polyether polyurethane onto 0.0508 cm (0.02 in) thick Eastar® 6763 PETG in such a way that the internal surface facing the contents to be packaged was of the PT9400. Each tray 10 was essentially a rectangle about 25.79 cm (10.156") long by 9.84 cm (3.875") wide. It had a 0.3175 cm (0.125") hinge groove which spanned its width located about 15.39 cm (6.06") from the short end distal from the angled shaft cavities. Each of the lower stem cavities was about 0.910 cm (0.3586") deep and 2.15 cm (0.8481") wide with radiused corners. The end of each lower stem cavity flared out to a width of 2.33 cm (0.92") at its end proximal to the body cavity 14. The distance from the end of this flare to the oblique wall 16 directly opposite the end of this flare was about 5.300 cm (2.0869"). Each of the angled shaft cavities 18 had a central axis at an angle of about 47° from the main axis of the tray component 10. The oblique wall 16 deviated from this main axis by about 47°.

## Claims

1. A rigid synthetic polymer package for medical articles, comprising:
(A) a rigid base (10) having an interior surface and an exterior surface, wherein said base (10) has at least two compartments (40; 50) including
(a) a first compartment (40) having
(i) a first interior surface (41) adapted for movement restraining contact with a first portion of a medical article, and
(ii) an opposing first exterior surface (42); and
(b) a second compartment (50) having
(i) a second interior surface (51) adapted for contact with a second portion of said medical article, and
(ii) an opposing second exterior surface (52);
wherein said first and second compartments (40; 50) of said base (10) are attached to each other by a connecting hinge (20) for manual movement of said first compartment (40) relative to the second compartment (50) to rotate said first interior surface (41) away from said second interior surface (51) and said connecting hinge (20) cannot be rotated to a position where said first interior surface (41) covers said second interior surface (51); and
(B) a cover (10) having at least two compartments (40; 50) including
(a) a first compartment (40) having
(i) a first interior surface (41) adapted for movement restraining contact with a first portion of a medical article, and
(ii) an opposing first exterior surface (42); and
(b) a second compartment (50) having
(i) a second interior surface (51) adapted for contact with a second portion of said medical article, and
(ii) an opposing second exterior surface (52);
said base (10) and cover (10) having closure means (30; 32) for connecting said base (10) and cover (10) together to provide a package for enclosing a medical article having a first portion and connected second portion; said closure means (30; 32) being manually openable for access and removal of said article;
wherein said first and second compartments (40; 50) of said cover (10) are attached to each other by a connecting hinge (20) which provides for manual movement of said first compartment (40) relative to the second compartment (50) to rotate said first interior surface (41) away from said second interior surface (51) and which cannot be rotated about said connecting hinge (20) to a position where said first interior surface (41) covers said second interior surface (51).

2. A package as defined in claim 1, wherein said base (10) is connected to said cover (10) by a peripheral hinge.

3. A package as defined in claim 1, wherein said base (10) and cover (10) are press fit together with said first and second compartments (40; 50) of said base (10) aligned with said first and second compartments (40; 50) of said cover (10).

4. A package as defined in any one of claims 1 to 3, wherein said interior surfaces of said base (10) and cover (10) comprise aromatic polyether polyurethane.

5. A package as defined in any one of claims 1 to 4, further comprising a medical article contained within said package and comprising a medical instrument, an implant, an orthopedic implant, a hip joint femoral stem, a shoulder implant, an implant with an abrasion sensitive coating, a cutting tool, or a fixation device for stabilizing a bone fracture.

6. A package as defined in any one of claims 1 to 5, wherein said first compartment (40) of said cover (10) has first and second cavities (14; 18) and said second compartment (50) has a cavity (12) with at least one channel connecting all three cavities (12; 14; 18).

7. A package as defined in any one of claims 1 to 5, wherein
said first compartment (40) of said cover (10) has first and second cavities (14; 18) and said second compartment (50) of said cover (10) has a cavity (12) with at least one channel connecting all three cavities (12; 14; 18);
said base (10) has a first compartment (40) having first and second cavities (14; 18) connected by an integrally formed hinge (20) to a second compartment (50) having a cavity (12) with at least one channel connecting all three cavities (12; 14; 18); and
at least one of said cover channels faces at least one of said base channels;
each channel having (i) a second compartment cavity (12) which is a longer hemi-tubular shape generally parallel to a long edge of said package; and (ii) a shorter hemi-tubular shaped second cavity (18) of said first compartment (40) which is at an acute angle to a longitudinal axis of said long edge;
wherein said first cavity (14) of said first compartment (40) connects said longer and shorter hemi-tubular cavities (12; 18) and said first cavity (14) has a significantly greater width parallel to a short edge of said package than said longer and shorter hemi-tubular cavities (12; 18) and with a wall in alignment with the longitudinal wall of said longer hemi-tubular cavity (12) closer to the periphery of said package;
wherein said closure means (30; 32) is adapted to reversibly lock said base (10) and cover (10) in a face to face configuration such that said longer and shorter hemi-tubular cavities (12; 18) form respective longer and shorter tubular cavities; and
wherein said cavities cooperate to establish a clearance which closely approximates a dimension of the largest member of a series of orthopedic stem components of different sizes.

## Patentansprüche

1. Starre synthetische Polymerverpackung für medizinische Artikel, umfassend:
(A) eine starre Basis (10) mit einer Innenfläche und einer Außenfläche, wobei die Basis (10) mindestens zwei Fächer (40; 50) hat, einschließlich
(a) eines ersten Fachs (40) mit
(i) einer ersten Innenfläche (41), die für bewegungshemmenden Kontakt mit einem ersten Abschnitt eines medizinischen Artikels ausgeführt ist, und
(ii) einer gegenüberliegenden ersten Außenfläche (42) und
(b) eines zweiten Fachs (50) mit
(i) einer zweiten Innenfläche (51), die für Kontakt mit einem zweiten Abschnitt des medizinischen Artikels ausgeführt ist, und
(ii) einer gegenüberliegenden zweiten Außenfläche (52), wobei das erste und das zweite Fach (40; 50) der Basis (10) über ein Verbindungsscharnier (20) für manuelle Bewegung des ersten Fachs (40) bezüglich des zweiten Fachs (50) aneinander angebracht sind, um die erste Innenfläche (41) von der zweiten Innenfläche (51) weg zu drehen, und das Verbindungsscharnier (20) nicht in eine Position gedreht werden kann, in der die erste Innenfläche (41) die zweite Innenfläche (51) abdeckt, und
(B) eine Abdeckung (10) mit mindestens zwei Fächern (40; 50), einschließlich
(a) eines ersten Fachs (40) mit
(i) einer ersten Innenfläche (41), die für bewegungshemmenden Kontakt mit einem ersten Abschnitt eines medizinischen Artikels ausgeführt ist, und
(ii) einer gegenüberliegenden ersten Außenfläche (42) und
(b) eines zweiten Fachs (50) mit
(i) einer zweiten Innenfläche (51), die für Kontakt mit einem zweiten Abschnitt des medizinischen Artikels ausgeführt ist, und
(ii) einer gegenüberliegenden zweiten Außenfläche (52), wobei die Basis (10) und die Abdeckung (10) ein Schließmittel (30; 32) haben, um die Basis (10) und die Abdeckung (10) miteinander zu verbinden, um eine Verpackung zum Umschließen eines medizinischen Artikels bereitzustellen, der einen ersten Abschnitt und einen verbundenen zweiten Abschnitt hat, wobei das Schließmittel (30; 32) zum Zugriff auf den und Entnahme des Artikels manuell geöffnet werden kann,
wobei das erste und das zweite Fach (40; 50) der Abdeckung (10) über ein Verbindungsscharnier (20) für manuelle Bewegung des ersten Fachs (40) bezüglich des zweiten Fachs (50) aneinander angebracht sind, um die erste Innenfläche (41) von der zweiten Innenfläche (51) weg zu drehen, und um das Verbindungsscharnier (20) nicht in eine Position gedreht werden können, in der die erste Innenfläche (41) die zweite Innenfläche (51) abdeckt.

2. Verpackung nach Anspruch 1, wobei die Basis (10) über ein Umfangsscharnier mit der Abdeckung (10) verbunden ist.

3. Verpackung nach Anspruch 1, wobei die Basis (10) und die Abdeckung (10) mit dem ersten und dem zweiten Fach (40; 50) der Basis (10) in Ausrichtung auf das erste und das zweite Fach (40; 50) der Abdeckung (10) zusammen eingepresst sind.

4. Verpackung nach einem der Ansprüche 1 bis 3, wobei die Innenflächen der Basis (10) und der Abdeckung (10) aromatisches Polyetherpolyurethan umfassen.

5. Verpackung nach einem der Ansprüche 1 bis 4, ferner umfassend einen in der Verpackung enthaltenen medizinischen Artikel, der ein medizinisches Instrument, ein Implantat, ein orthopädisches Implantat, einen Hüftgelenk-Femurschaft, ein Schulterimplantat, ein Implantat mit einer abriebempfindlichen Beschichtung, ein Schneidwerkzeug oder eine Fixationsvorrichtung zum Stabilisieren einer Knochenfraktur umfasst.

6. Verpackung nach einem der Ansprüche 1 bis 5, wobei das erste Fach (40) der Abdeckung (10) einen ersten und einen zweiten Hohlraum (14; 18) hat und das zweite Fach (50) einen Hohlraum (12) mit mindestens einem Kanal hat, der alle drei Hohlräume (12; 14; 18) verbindet.

7. Verpackung nach einem der Ansprüche 1 bis 5, wobei das erste Fach (40) der Abdeckung (10) einen ersten und einen zweiten Hohlraum (14; 18) hat und das zweite Fach (50) der Abdeckung (10) einen Hohlraum (12) mit mindestens einem Kanal hat, der alle drei Hohlräume (12; 14; 18) verbindet,
die Basis (10) ein erstes Fach (40) mit einem ersten und einem zweiten Hohlraum (14; 18) hat, das über ein integral ausgebildetes Scharnier (20) mit einem zweiten Fach (50) verbunden ist, das einen Hohlraum (12) mit mindestens einem Kanal hat, der alle drei Hohlräume (12; 14; 18) verbindet, und
mindestens einer der Abdeckungskanäle mindestens einem der Basiskanäle zugewandt ist,
jeder Kanal (i) einen zweiten Fachhohlraum (12) hat, der eine längere halbrohrförmige Gestalt hat, die allgemein parallel zu einem langen Rand der Verpackung verläuft, und (ii) einen kürzeren halbrohrförmigen zweiten Hohlraum (18) des ersten Fachs (40), der in einem spitzen Winkel zu einer Längsachse des langen Rands verläuft,
wobei der erste Hohlraum (14) des ersten Fachs (40) den längeren und den kürzeren halbrohrförmigen Hohlraum (12; 18) verbindet und der erste Hohlraum (14) eine wesentlich größere Breite parallel zu einem kurzen Rand der Verpackung hat als der längere und der kürzere halbrohrförmige Hohlraum (12; 18) und eine Wand, die auf die Längswand des längeren halbrohrförmigen Hohlraums (12) näher an dem Umfang der Verpackung ausgerichtet ist,
wobei das Schließmittel (30; 32) dazu ausgeführt ist, die Basis (10) und die Abdeckung (10) in einer gegenübergestellten Konfiguration reversibel zu verriegeln, so dass der längere und der kürzere halbrohrförmige Hohlraum (12; 18) längere bzw. kürzere rohrförmige Hohlräume bilden, und
wobei die Hohlräume zusammenwirken, um einen Freiraum zu schaffen, der einer Abmessung der größten Glieds einer Reihe von orthopädischen Schaftkomponenten unterschiedlicher Größe nahekommt.

## Revendications

1. Emballage polymère synthétique rigide pour articles médicaux, comprenant :
(A) une base rigide (10) ayant une surface intérieure et une surface extérieure, ladite base (10) ayant au moins deux compartiments (40 ; 50) comprenant
(a) un premier compartiment (40) ayant
(i) une première surface intérieure (41) adaptée pour être mise en contact de retenue de mouvement avec une première partie d'un article médical, et
(ii) une première surface extérieure opposée (42) ; et
(b) un second compartiment (50) ayant
(i) une seconde surface intérieure (51) adaptée pour être mis en contact avec une seconde partie dudit article médical, et
(ii) une seconde surface extérieure opposée (52) ; lesdits premier et second compartiments (40, 50) de ladite base (10) étant fixés l'un à l'autre par une charnière de liaison (20) pour un mouvement manuel dudit premier compartiment (40) par rapport au second compartiment (50) pour faire tourner ladite première surface intérieure (41) à l'opposé de ladite seconde surface intérieure (51) et ladite charnière de liaison (20) ne pouvant pas être tournée dans une position où ladite première surface intérieure (41) recouvre ladite seconde surface intérieure (51) ; et
(B) un couvercle (10) ayant au moins deux compartiments (40 ;50) comprenant
(a) un premier compartiment (40) ayant
(i) une première surface intérieure (41) adaptée pour être mise en contact de retenue de mouvement avec une première partie d'un article médical, et
(ii) une première surface extérieure opposée (42) ; et
(b) un second compartiment (50) ayant
(i) une seconde surface intérieure (51) adaptée pour être mis en contact avec une seconde partie dudit article médical, et
(ii) une seconde surface extérieure opposée (52) ; ladite base (10) et ledit couvercle (10) ayant des moyens de fermeture (30, 32) pour relier ladite base (10) et ledit couvercle (10) l'un à l'autre pour fournir un emballage destiné à contenir un article médical ayant une première partie et une seconde partie reliée ; lesdits moyens de fermeture (30 ; 32) pouvant être ouverts manuellement pour l'accès et le retrait dudit article ;
lesdits premier et second compartiments (40 ; 50) dudit couvercle (10) étant fixés l'un à l'autre par une charnière de liaison (20) qui permet un mouvement manuel dudit premier compartiment (40) par rapport au second compartiment (50) pour faire tourner ladite première surface intérieure (41) à l'opposé de ladite seconde surface intérieure (51) et qui ne peut pas être tournée autour de ladite charnière de liaison (20) jusqu'à une position où ladite première surface intérieure (41) recouvre ladite seconde surface intérieure (51).

2. Emballage selon la revendication 1, ladite base (10) étant reliée audit couvercle (10) par une charnière périphérique.

3. Emballage selon la revendication 1, ladite base (10) et ledit couvercle (10) étant ajustés ensemble par pression, avec lesdits premier et second compartiments (40; 50) de ladite base (10) alignés avec lesdits premier et second compartiments (40 ; 50) dudit couvercle (10).

4. Emballage selon l'une quelconque des revendications 1 à 3, lesdites surfaces intérieures de ladite base (10) et dudit couvercle (10) comprenant du polyuréthane de polyéther aromatique.

5. Emballage selon l'une quelconque des revendications 1 à 4, comprenant en outre un article médical contenu à l'intérieur dudit emballage et comprenant un instrument médical, un implant, un implant orthopédique, une tige fémorale pour l'articulation de la hanche, un implant d'épaule, un implant avec un revêtement sensible à l'abrasion, un outil de coupe ou un dispositif de fixation pour stabiliser une fracture osseuse.

6. Emballage selon l'une quelconque des revendications 1 à 5, ledit premier compartiment (40) dudit couvercle (10) ayant des première et seconde cavités (14; 18) et ledit second compartiment (50) ayant une cavité (12) avec au moins un canal reliant chacune des trois cavités (12 ; 14 ; 18).

7. Emballage selon l'une quelconque des revendications 1 à 5, ledit premier compartiment (40) dudit couvercle (10) ayant des première et seconde cavités (14 ; 18) et
ledit second compartiment (50) dudit couvercle (10) ayant une cavité (12) avec au moins un canal reliant chacune des trois cavités (12 ; 14 ; 18) ;
ladite base (10) ayant un premier compartiment (40) ayant des première et seconde cavités (14 ; 18) reliées par une charnière formée d'un seul tenant(20) à un second compartiment (50) ayant une cavité (12) avec au moins un canal reliant chacune des trois cavités (12 ; 14 ; 18) ; et
au moins l'un desdits canaux de couverture étant orienté vers au moins l'un desdits canaux de base ; chaque canal ayant (i) une seconde cavité de compartiment (12) qui est de forme hémi-tubulaire plus longue généralement parallèle à un bord long dudit emballage ; et (ii) une seconde cavité de forme hémi-tubulaire plus courte (18) dudit premier compartiment (40) qui forme un angle aigu avec un axe longitudinal dudit bord long ;
ladite première cavité (14) dudit premier compartiment (40) reliant lesdites cavités hémi-tubulaires plus longues et plus courtes (12 ; 18) et ladite première cavité (14) ayant une largeur sensiblement plus grande parallèlement à un bord court dudit emballage que lesdites cavités hémi-tubulaires plus longues et plus courtes (12; 18) et avec une paroi en alignement avec la paroi longitudinale de ladite cavité hémi-tubulaire plus longue (12) plus proche de la périphérie dudit emballage;
ledit moyen de fermeture (30 ; 32) étant adapté pour verrouiller de manière réversible ladite base (10) et ledit couvercle (10) dans une configuration face à face de telle sorte que lesdites cavités hémi-tubulaires plus longues et plus courtes (12; 18) forment des cavités tubulaires respectives plus longues et plus courtes ; et
lesdites cavités coopérant pour établir un dégagement qui se rapproche étroitement d'une dimension de l'élément le plus grand d'une série de composants de tiges orthopédiques de différentes tailles.
